(19) Europäisches Patentamt
European Patent Office
Office européen des brevets

(11) EP 2 380 553 A2

(12) **EUROPEAN PATENT APPLICATION**

(43) Date of publication:
**26.10.2011 Bulletin 2011/43**

(21) Application number: **10015460.8**

(22) Date of filing: **09.12.2010**

(51) Int Cl.:
***A61K 8/14*** *(2006.01)* ***A61K 8/55*** *(2006.01)*
***A61K 8/73*** *(2006.01)* ***A61K 8/81*** *(2006.01)*
***A61K 8/84*** *(2006.01)* ***A61Q 5/00*** *(2006.01)*
***A61Q 19/00*** *(2006.01)* ***B82Y 5/00*** *(2011.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **07.01.2010 AR P100100026**

(71) Applicant: **Lo Riggio, Victor Pascual**
**(1754) Sant Justo-Pcia. de Bs. As. (AR)**

(72) Inventor: **Lo Riggio, Victor Pascual**
**(1754) Sant Justo-Pcia. de Bs. As. (AR)**

(74) Representative: **Pereira Toña, Maria Irache**
**Accions en Patents I Marques Padulles, S.L.**
**C/Enric Granados, 21, Pral, 4a,**
**08007 Barcelona (ES)**

(54) **Nanovector with positive charge substantive for hair and skin**

(57) "NANOVECTOR WITH POSITIVE CHARGE SUBSTANTIVE FOR HAIR AND SKIN", to be applied directly on the skin or hair, or to be added to the preparation of a cosmetic or *dermopharmaceutical* compositions in order to enhance the properties of said products; comprising empty particles dispersed in a water-based medium of the range of the nanometers; those particles have mainly the following components: a phospholipids membrane of any order and composition, and one or more quaternary polymers that provide it with a superficial positive charge, both components may be found in any concentration combination.

RESULTS

CURVE OF THE BREAK RESISTANCE:

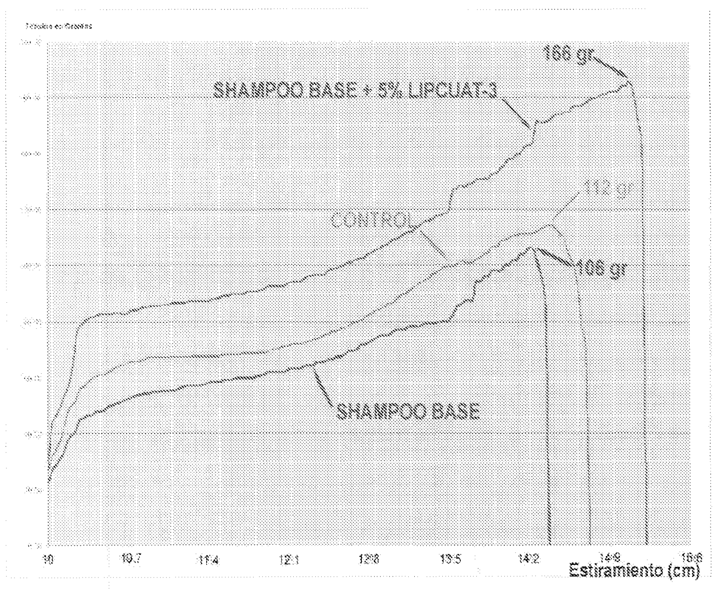


FIGURE 9: in the graphic we can appreciate that the hair treated with LIPCUAT is more resistant to tension (more charge) before it breaks, the product improves the resistance of the capillary fiber in comparison to the hair treated with the base shampoo.

EP 2 380 553 A2

**Description**

## TECHNICAL FIELD

This invention relates to a nanovector with positive charge substantive to hair and skin. And, more precisely, it relates to these nanovectors as empty, spherical particles that consist of a phospholipid membrane combined with one or more cationic polymers of the size of a nanometer (less than a micron). These particles are found dispersed in a water-based medium which consists of a fluid of low, medium or high viscosity level, generally homogeneous. The composition can be applied directly on the skin or hair, or it may be added to the preparation of a cosmetic or *dermopharmaceutical* composition containing from about 0.05 to 50% in order to enhance the properties of said products.

## BACKGROUND OF THE INVENTION

Up to the present, there have been several patents and papers published, such us:

USA Patent 5.328.628, of July 12, 1994.

Revista Dermatologia Venzolana [Dermatology Magazine of Venezuela], Vol. 33, N° 1, Venezuela, year 1995.

Dra. Beatriz Clares Naveros' Doctoral Thesis, Granada, Spain, year 2003.

Journal of Oleo Science N° 56 page. 35 to 42, Japan Oli Chemists' Society, Japan, year 2007.

All of these publications deal with the formulation of the liposome formulas, its manufacture and application in cosmetics and/or *dermopharmaceutical* compositions. But, the object of the present invention has not been dealt with in any of these publications. This invention relates to a product that can be applied directly on the skin or on the hair or added to the preparation of a cosmetic or *dermopharmaceutical* compositions in order to enhance the properties of said products, such as to have a great substantivity to hair and skin; having the capacity of penetrating the capillary fiber and the corneous stratum; being able to protect and stabilize the carried actives; in the case of shampoos, the capacity to enhance the substantivity of the carried actives, thus creating a protective layer over the capillary fiber; to provide a velvety feeling when in contact with the skin or hair; the capacity of making the hair soft, shiny and manageable, making it more elastic and resistant to traction; to moist and lubricate the hair's surface; to reduce the irritability of the surfactant agents; to enable the fat-soluble substances to be added without the need to emulsifying them.

## DETAILED DESCRIPTION OF THE INVENTION

The main object of this invention is a "NANOVECTOR WITH POSITIVE CHARGE SUBSTANTIVE FOR HAIR AND SKIN", these type of nanovector are to be applied directly on the skin or hair, or to be added to the preparation of a cosmetic or *dermopharmaceutical* composition in order to enhance the properties of said products; characterized by having empty particles of the size of a nanometer dispersed in a water-based medium; these particles mainly having two components: a phospholipid membrane of any order and composition, and more than one or several quaternary polymers which provide a superficial positive charge, both components can be found in any concentration combination.

The applied phospholipids can be from vegetable (mainly soy lethitin) as well as from animal (nervous tissue, eggs, etc.) sources, or synthetic.

The phospholipids from vegetable source are generally composed by phosphatidylcholine, phosphatidylethanolamine, phosphatidylinositol, phosphatidylserine, fatty acids, etc.

The concentration of the phospholipids that are found on the membrane can go from 1 to 30%, more preferably from 5 to 15%.

Among the quaternary polymers we can find the Polyquaternium 16 (from the brand LUVIQUAT of the company BASF) formed from methylvinylimidazolium (QVI) chloride and vinylpyrrolidone (VP), We may also find the Polyquaternium 7, which is very well known for its application in shampoos and hair conditioners. And, the Polyquaterniums 1, 2, 6, 10, 22, 28, 32, 39, 46 and 47. The concentration of the quaternary polymer(s) can go from 1 to 40%, more preferably from 5 to 15%.

The nanovectors of this invention carry within themselves water-soluble substances (hydrophilic molecules: salts, proteins, amino acids, vitamins, vegetable extracts, etc.) and within its membranes oil-soluble substances can be found (hydrophobic molecules: oils, silicones, fats, high molecular weight polymers, vitamin A, vitamin E, flavonoids, etc.)

The membrane can have antioxidant substances, butyl hydroxy toluene (BHT), butyl hydroxy anisole (BHA), vitamin E, vitamin C, etc., in order to avoid the membrane and the carried actives to oxidize.

The nanovectors of this invention as well as the liposomes can be unilamellar (can have only one layer of phospholipids), multilamellar (can have several layers of phospholipids), or oligolamellar (can have the combination of the previous options).

These dispersions, which have a superficial positive charge (+), have a great affinity with the hair and skin, thus, they stick strongly to them. This phenomenon is known as substantivity. The sizes of the particles are generally in a range below 1 micron (1,000 nanometers), more preferably, below the 500 nanometers.

In order to achieve a better physic stability as well as hair and corneum stratum penetration, the size is reduced below the 500 nanometers. This is achieved by a process called micro fluidification, by means of which the particles undergo abrupt changes of pressure and speed, literally exploding into much smaller particles.

Other additives such as co-surfactants can be added in order to enhance the stability of the encapsulated active or to reduce the size of the particles. Among these, the most commonly used polysorbates are the TWEEN 20 and TWEEN 80.

The nanovectors of this invention arc high performance vehicles that also carry oriented active components, enabling a great adhesion to capillary fiber and skin keratin.

The membrane of these nanovectors may have antioxidant substances and/or sun screens that protect the encapsulated active(s).

Conservatives: the preservatives are necessary in order to maintain the product in good microbiologic conditions. The nanovectors of this invention are compatible with any type of preservative: imidazolidinyl) urea, propylparaben, methylparaben, DMDM hydantoin, fenoxietanol, benzalkonium chloride, Kathon CG (Methylchloroisothiazolinone and Methylisothiazolinone), etc.

The nanovectors of this invention have the following cosmetic properties:

1) They have a great substantivity to hair and skin due to its superficial positive charge density (+).

2) Due to their small size (at the nanometer level) they have the capacity of penetrating the capillary fiber and the corneum stratus.

3) They protect and stabilize the actives they carry.

4) In the shampoo formulas, they enhance the substantivity of the actives they carry in reference to those added individually.

5) They create a protective layer on the capillary fiber.

6) They provide a velvety feeling when in contact with the skin or hair.

7) In the case of damaged hair, it conditions it and makes it brighter and softer.

8) They make it more manageable, avoiding the hair to break.

9) They make it more elastic and resistant to traction.

10) They help to reduce the irritability caused by formulas containing surfactant agents.

11) They moisturize and lubricate the hair surface.

12) They allow us to add fat soluble substances without having the need of emulsifying them with surfactant agents that are hazardous to the skin and hair, for example: vitamin E, vitamin A, coenzyme Q10.

13) The properties of the actives carried by the nanovectors must be added to the properties herein above mentioned.

The characteristics herein above mentioned constitute the object of this invention that is highly recommended to make: hair conditioners, leave on conditioners, lotions, creams, gels, emulsions, soaps, and any other cosmetic and dermopharmaceutical compositions. It is incorporated in an easy and fast way in those formulas that need to be shaken and in those that undergo temperatures under 60 degrees centigrades.

The studies, tests and applications carried out in relationship to the product of this invention (from now on mentioned as LIPCUAT) are found herein below:

1) STUDY OF THE SUBSTANTTVITY TO HAIR

Foundations:

The hair absorption of the cationic components may be evident by the so called "Rubine Dye Test", due to the fact that said coloring has been removed from the market, a similar test is carried out using the coloring Pyrazol Fast Red 7BSW (Direct Red 80).

This coloring specifically reacts with cationic components, showing its deposition on the hair due to the development of its intense scarlet color.

The preparation of the reactive:

Weight 250 mg of Direct Red 80 and dissolve it in 50 ml of distilled water, leveling its pH to 3.50 with sulfuric acid.

Hair preparation:

The blonde hair is immerged in hydrogen peroxide V20 (200 ml) and afterwards 3 ml of ammoniac is added. The hair is left to get bleached for 60 minutes. Afterwards the hair is washed with running water and left to dry.

Procedure:

1. Approx.. 500 mg of bleached hair is weighted.

2. The hair immersed in ethanol 96°; afterwards left 10 minutes with agitation and then dried.

3. Once it is dried, it is immersed in the product to be tested in the required levels for three minutes (in the case of the LIPCUAT, they applied 1% of the solution).

4. The hair is rinsed carefully with running water.

5. The hair is washed with a shampoo that does not contain quaternaries.

6. It is afterwards rinsed with running water.

7. Immediately and while still wet.

8. The hair is immersed in the coloring during 5 minutes at a temperature of 40°C.

9. Then, the hair is taken out of the container and rinsed very well with water and the intensity of the red coloring is examined.

10. This procedure is also carried out with a lock of control hair, which is immersed in the equivalent of distilled water. Then, the steps 3 to 5 are carried out.

FIGURE 1: Hair treated with LIPCUAT. In this figure you can appreciate that the red coloring adheres to the hair, thus the presence of the cationic components on the capillary surface is confirmed.
FIGURE 2: Control hair: not treated with LIPCUAT. The coloring does not adhere to the capillary fiber. The color of the hair is not altered due to the fact that there are no cationic components over the capillary fiber, thus the red coloring does not adhere.

CONCLUSION: THE LIPCUAT HAS A HIGH ABILITY TO STICK TO THE HAIR AND IS RESISTENT TO RINSING.

2) MEASUREMENT OF THE SIZE OF THE PARTICLES BY MEANS OF A LASER LIGHT SCATTERING

Importance of the measurement: the ability of the particles to penetrate the capillary fiber is limited by its size. The smaller they are, the easier it will be for them to reach the micro-injuries or the porous areas of the damaged hair. In those areas is where the carried actives release its charge, Those actives are in charge of reconstruct and repair de

capillary fiber.

Description:

A measurement of the distribution of the size of the particles is determined by LASER LIGHT SCATTERING.

Applied equipment: MASTERSIZER 2000

Sample: 100 ml of LIPCUAT.

Results of the analysis: In the attached report we can find the following information:

a) All of the particles had a size below the 0.275 microns.
b) The smaller particle had a size of 0.06 microns.
c) The 50% of the particles had a size below 0.105 microns.
d) The average particle size is of 0.108 microns.

Result: the microfluidification process applied in order to produce the LIPCUAT enables us to obtain an ideal particle size and distribution in order to enhance the penetration and performance of the product on the capillary fiber.

3) LIPCUAT CRYOFRACTURE

In the FIGURE 3 (PICTURE) we can appreciate the spherical structure, even the emptiness of the nanoparticle and the size below the 300 nanometers in comparison with the reference bar.

FIGURE 3. Microscopic view.

Technique: Transmission electron microscopy (TEM) (Zeiss EM902), afterwards a photograph of the cryofracture is taken with a digital Nikkon camera. This study is carried out in the *Universidad Nacional de Cuyo* [National University of the Cuyo]. Faculty of Medical Science. *IHEM CONICET* [Histology and Embryology Institute of the city of endoza]. Av. Libertador 80. Centro Universitario. Parque Oral. San Martin (5500) Mendoza. Argentina.

Scanning electron microscope examination.

FIGURE 4: Scanning electron microscope examination. Carried out in the Faculty of Odontology.

The picture illustrates the Nanovectors (+) micro-fluidificated. As we compare the size of them with the reference bar (1 micron) we can appreciate that the average size is below the 300 nanometers.

4) STABLISHING THE LIPCUAT CHARGE BY MEANS OF ELECTROPHORESIS.

Procedure: a run with electrophoresis of agarose gel is carried out. Several LIPOSOMES with negative superficial charge (-) are scattered in the center and 3 lanes with LIPCUAT with positive superficial charge (+).

[On the top of the picture it reads: LIPOSOMES WITH NEGATIVE SUPERFICIAL CHARGE. On the bottom of the picture it reads: LIPCUAT WITH POSITIVE SUPERFICIAL CHARGE]

FIGURE 5. From this picture we can appreciate that the product object of this invention (LIPCUAT) migrates towards the anode charge (-). We also can appreciate that the migration occurs in an opposite direction of that of the LIPOSOMES with negative superficial charge (+). Thus, we can affirm that LIPCUAT has a net positive superficial charge (+).

5) SOLAR PROTECTION OF THE LIPCUAT TESTED ON NATURAL HAIR

Introduction: within its structure, LIPCUAT has a high performance solar filter (octyl methoxycinnamate) that protects the hair from the UV rays. Thanks to it substantivity, the positive charge (+) of the nanovectors adheres them strongly to the hair surface protecting the hair from solar rays.

Objective: the object of this test is to demonstrate the protective ability and substantivity of the LIPCUAT,thus an excellent protective layer against solar radiations is created. Materials: Equipment applied:

Digital microscope ESPA, Magnifier 60X.

SalonDry Control Philips hair drier.

Analytic scales.

Solvents and standards applied:

Carbomer, sodium hydroxide, Kathon CG: methylchloroisothiazolinone (and) methylisothiazolinone.

Neutral detergent.

Natural hair.

Procedure:

1- Two gels with the following formulas are prepared:

Gel base: water 99.2% - Carbomer 0.7% - sodium hydroxide qs pH 6-Kathon 0.1%

Gel with 5% of LIPCUAT (octal methoxycinnmate, panthenol, phenyl trimethicone): water 94.2% - Carbomer 0.7% - sodium hydroxide qs pH 6- Kathon 0.1% - LIPCUAT 5%.

2- Three locks of healthy, natural hair without chemical treatment are chosen. The hair belongs to a 27 year old woman; the locks have been removed from the external layer of back of the neck.

3- The three locks weight 350 mg each and they are fastened by adhesive tape.

4- The three locks are washed with natural detergent and rinsed with plenty of water.

5- The gel with a 5% of LIPCUAT is applied to one of the locks as follows: a) it is washed with a natural detergent, massage it for 2 minutes and then rinsed with plenty of water; b) 2.5 gr of gel with 5% of LIPCUAT and the lock, still humid and located in the palm of the hand, is massaged with both hands; c) the gel with a 5% of LIPCUAT is spread over all the surface of the lock using the fingers, making sure all the surface is covered; d) the lock is dried with the Philips drier with hot and dry air, at the maximum temperature during 5 minutes.

6- The base of the gel added to the other lock will follow the steps a) to d) herein above mentioned.

7- The third lock is dried with the Philips hair drier with hot dry air at the maximum temperature during 5 minutes without adding anything to it.

8- The three locks arc tagged as follows:

Lock - Exposed to the sun - with gel with a 5% of LIPCUAT.

Lock - Exposed to the sun - with gel base.

Lock - without being exposed to the sun.

9- The two locks tagged as "exposed to the sun" are placed in a first floor balcony, on the cast side on a white surface.

10- Both locks are left there from 8:30 a.m. until 5:50 p.m.

11- Each day and for a period of 21 days the locks are washed, the respective products are applied and they are dried as detailed herein above.

After 21 days, the last centimeters of the ends of each hair are observed with the digital microscope. Pictures of each one are taken, which are found herein below:

Lock - Exposed to the sun - with gel with a 5% of LIPCUAT.

FIGURE 6: the hair protected with LIPCUAT does not show any modification in its cuticle, it is soft and has no imperfections. The hair maintains its elasticity, When touched, you have a soft feeling.

Lock - Exposed to the sun - with gel base.

FIGURE 7: the hair with gel base, without any protection, is heavily affected by the solar rays, its cuticle is destroyed and its body is cracked as if it had been exposed to high temperatures or even burnt. The hair is fragile, rough and when touched, you have an unpleasant feeling.

Lock - without being exposed to the sun.

FIGURE 8: the hair has not been exposed to the sun and you can appreciate that its cuticle is intact, slightly lifted. When touched, it feels less rough that the hair without protection, but, it feels rougher than the lock protected by LIPCUAT.

CONCLUSION: the LIPCUAT is effective for hair protection, specially its ends, from solar radiation. It also conditions, maintains and improves the elasticity of the hair, it makes it softer and brighter at the same time.

6) TEST ON THE HAIR TRACTION RESISTENCE WHEN TREATED WITH LIPCUAT

Introduction: daily wash, chemical treatments, UV rays and time weaken the capillary fiber. This leads to the increase of hair loss, reduces its density and elasticity, etc.

The measurements carried out confirm that the more deteriorated and more mistreated the hair is the less resistance it has to traction.

Objective: to determine the ability of LIPCUAT to enhance the traction resistance of the hair once it is added to a shampoo.

Method: the hair is stretched until it is broken while its traction resistance is measured by means of a digital dynamometer- Locks rinsed with water are compared with those washed with shampoo and those washed with shampoo with LIPCUAT.

Materials used:

AHSIM A&L digital dynamometer.

Philips Salon Dry Control hair dryer.

Needed amount of LIPCUAT.

Sodium lauryl sulfate (25% Etoxylated)

PROCEDURE:

1- Prepare 2 shampoos with the following formulas:

Shampoo A (shampoo base): 75% of distilled water - 25% of sodium lauryl sulfate etoxylated.

Shampoo B (shampoo containing a 5% of LIPCUAT): 70% of distilled water - 25% of sodium lauryl sulfate etoxylated - 5% of LIPCUAT.

2- Weight 6 locks of 350 mg of the same natural hair, which are no less than 15 cm long.
3- Tag the locks as follows:

2 locks: SHAMPOO A (SHAMPOO BASE).

2 locks: SHAMPOO B (SHAMPOO WITH A 5% OF LIPCUAT).

2 locks: CONTROL HAIR (DISTILLED WATER).

4- Wash each lock 15 times with its corresponding shampoo, or in the case of the control hair, with distilled water, as follows: a) wet with warm water, afterwards 0.5 gr of SHAMPOO (whichever applies), then massaged with both hands for 2 minutes and finally rinsed with abundant water; b) the locks are dried with hot air by means of a hair dryer in its maximum temperature for 5 minutes.
Note: the CONTROL HAIR locks are washed 15 times as described herein above in items a) and b) without shampoo.
5- Six hairs of every lock are taken and its resistance to tension and stretching is measured in each case and the final results are averaged out.
NOTE: the initial length of the hair before the test is carried out is of 10 centimeters.

RESULTS

FIGURE 9: in the graphic we can appreciate that the hair treated with LIPCUAT is more resistant to tension (more charge) before it breaks, the product improves the resistance of the capillary fiber in comparison to the hair treated with the base shampoo.
FIGURE 10: in the graph bar it is clearly shown that the resistance is improved in the hair treated with LIPCUAT compared to the control hair. We can also appreciate how the resistance is reduced with the base shampoo compared to the control.
CONCLUSION: The hair resistance to traction is enhanced by LIPCUAT. The capillary fiber becomes more resistant and it breaks less. From these results we can infer that LIPCUAT reduces hair loss during combing.

7) <u>TEST ON THE SUBSTANTIVITY AND ON THE LIPCUAT LAYER FORMATION.</u>

Objetive of the test:

To determine the LIPCUAT's substantivity (ability to adhere to the structure) with the hair, even after it has been rinsed.

To verify the ability to penetrate though the capillary structure pores.

To confirm the formation of a film over the surface as a consequence of the particles fusion.

Method: the scanning electron microscope (SEM) is used in order to observe the capillary fiber and the LIPCUAT in different conditions,

Materials:

Used equipment:

Scanning electron microscope ESEM SEM S30 from PHILIPS

Place: *SEGEMAR (INTI)* [Geologicaland Mining Argentine Service] / Argentine Republic.

Digital Micrometer (Digimess)

Natural hair without chemical treatment (of a 28 year old woman)

Needed amount of LIPCUAT.

Procedure:
a)- Selection of hairs to study.By means of a digital micrometer natural hairs without chemical treatment of similar diameter are selected.

| HAIR | A | B | C | D |
|---|---|---|---|---|
| DIAMETER (mm) | 0.075 | 0.074 | 0.075 | 0.075 |

b) The following tests are performed on the selected hairs as follows:

Han Λ. Without treatment (Control).

Test 1: Hair B· The hair is immersed in LIPCUAT for 5 minutes: afterwards it is left to drain and dry outdoors.

test 2: Hair C: The hair is immersed in a solution with a 5% of LIPCUAT for 5 minutes, afterwards it is left to drain and dry outdoors.

Test 3: Hair D: The hair is immersed in a solution with 1% of LIPCUAT for 5 minutes, afterwards it is rinsed with plenty of water and let to drain and dry outdoors.

c)- Every hair is put over its assigned glass Petri dish and they are tagged as follows Hair A/ Hair B/ Hair C/ Hair D.
d)- After 24 hours, the treated and untreated hairs arc located on the glass dishes and when dry, a thin layer of gold is applied to them (metalize) in order to be observed through the electron microscope
e)- The samples are observed through the scanning electron microscope and digital photographs are taken. Test 4· A drop of LIPCUAT is put on the glass dish and it is let to dry at room temperature outdoors
After 24 hours, the treated and untreated hairs are located on the glass dishes and when dry, a thin layer of gold is applied to them (metalize) in order to be observed through the electron microscope.
Digital photographs arc taken.

Test 1.

Hair A: without treatment.
Hair B: The hair is immersed in LIPCUAT for 5 minutes; it is let to drain and dry outdoors.

The hairs are observed 24 hours later with the scanning electron microscope SEM.
FIGURE 11: Hair A (control) Hair B (treated with LIPCUAT)

FIGURE 12: Hair A (cuticle detail) Hair 13 (cuticle detail)

Test 2

Hair A: Without treatment.
Hair C: The hair is immersed in a solution with 5% of LIPCUAT for 5 minutes, afterwards it is left to drain and dry outdoors.

The hairs are observed 24 hours later with the scanning electron microscope SEM.
FIGURE 13: Hair A (cuticle detail) Hair C (cuticle detail)

Test 3:

Hair A: without treatment.

Hair D: The hair is immersed in a solution with 1% of LIPCUAT for 5 minutes; afterwards it is rinsed with plenty of water and let to drain and dry outdoors.

The hairs are observed 24 hours later with the scanning electron microscope SEM.
FIGURE 14: Hair A (control) Hair D (treated with LIPCUAT)
FIGURE 15: Hair A (cuticle detail) Hair D (cuticle detail)
FIGURE 16: Hair D (cuticle detail)

Test 4:

A drop of LIPCUAT is put on the glass dish and is left to dry at room temperature.
The drop is observed 24 hours later with the scanning electron microscope SEM.
FIGURE 17: Fusioned LIPCUAT particles creating a film on the glass dish.

RESULTS:

Figure 11: hairs A and B. We can appreciate that there is a film over hair B.
Also, the hair B treated with LIPCUAT has a larger diameter.
This phenomenon may be due to the fact that the fiber get wider due to the moisturizing effect and also due to the layer that covers the hair.
Figure 12: we can appreciate that in the cuticle detail in hair A and B, the latter has a layer that acts as filler.
The empty spaces that are found between the flakes on the cuticle of hair A are deeper that those found in hair B.
This happens due to the fact that the film fills the empty spaces.
Figure 13: we can appreciate the cuticle detail in hair A, which is untreated,and C, which is treated with a solution with 1% of LIPCUAT.
The slots on the flakes found on the cuticle of hair A are beeper than those found on the cuticle of hair C.
This phenomenon is due to the layer formed on the hair as a consequence of the LIPCUAT particle fusion.
Even though the filler effect is less compared to that one in Figure 12, we can appreciate that there is a thin layer as a consequence of the LIPCUAT membranes' fusion.
Figure 14: in the photograph we can appreciate the details of the simple of hair A, which had no treatment, and the sample of hair D, which is treated with LIPCUAT and afterwards rinsed with plenty of water.
Spherical and avoids structures, the LIPCUAT particles, can be appreciated on the surface of hair D.
In this figure, we are looking for the deteriorated areas on hair D in order to observed the particles behavior in those areas (see Figure 15).
Figure 15: in this picture we can appreciate the cuticle details of hair A, which is untreated, and hair D, which is treated with LIPCUAT and afterwards rinsed with plenty of water.
Spherical and ovoids structures, the LIPCUAT particles, can be appreciated on the surface of hair D.
We can also see that there are several crowded particles on the empty spaces found between the flakes functioning as fillers (see area marked with a circle).
Figure 16: this is an enlargement (see reference line 2 nm) of the detail of the cuticle flakes on hair D, which is treated with LIPCUAT, afterwards rinsed. We can find herein spherical structures sticked to the surface.
These particles are not found on hair A, which had no treatment.
Figure 17: after 24 hours, we can appreciate that the undiluted LIPCUAT particles, once dry, have fusioned, thereby creating a continuous film on the glass dish.
This behavior may explain the reason why we can observe a continuous film over the surface of the hairs treated with LIPCUAT, hairs B and C, once dry and after 24 hours, instead of observing particles.

CONCLUSIONS:

a)-After 24 hours of applying LIPCUAT to the hair, once it is dry, the product creates a continuous film due to the fusion of its particles.

b)-LIPCUAT sticks to the hair, thereby making it thicker.

c)-The LIPCUAT particles have a substantivity to hair, thus, they stick to it even after it has been rinsed with plenty of water.

d)-The LIPCUAT particles crowd together in empty spaces found under the flakes of the cuticle, thus functioning as filler even after the hair is rinsed.

<u>8) TEST ON THE SKIN RASH PROVOQUED BY THE ANIONIC SURFACTANT.</u>

Introduction: The sodium dodecyl sulfate (SDS or NaDS) ($C_{12}H_{25}NaO_4S$), also known as sodium lauryl sulfate (SLS) is an anionic surfactant that is used for amenities, such as tooth paste, shampoos and bath soaps.
As in most of surfactant detergents, soaps included, the SDS attacks hair and skin, and eliminates their natural lubrication, thereby provoquing dermatitis, skin rash and the irritation of the eyes.
We used the SDS as the irritating substance due to the fact that it is one of the most applied surfactants and the one that produces more reactions on the skin.
Test 1 is carried out in 6 people, 3 men and 3 women between 25 and 52 years old.
Materials:

FINN chambers an SDS absorbing discs from SIGMA.
3 Solutions are prepared:

1) A solution with 2.5% of SDS (sodium lauryl sulfate) in distilled water (positive control).

2) A solution with 2.5% SDS (sodium lauryl sulfate) in distilled water + a 0.5% of

LIPCUAT-4

3) A solution with 2.5% SDS (sodium lauryl sulfate) in distilled water + a 2% of LIPCUAT-4

Method:

a) Clean the area to be tested with a solution containing a 70% of ethanol.

b) Number the FINN chambers.

c) Open the FINN chamber and place in it an absorbing disc.

e) Apply 0.0.5 ml. of the solutions 1, 2 and 3 respectively in each disc with a syringe.

d) Place the 3 patches 1, 2 and 3 on the forearm.

FIGURE 18: FINN chambers on the forearm.

e) These are left for 24 hours and then removed.

f) The area is cleaned with a solution containing a 70% of ethanol.

g) After 30 minutes, the tested areas are evaluated.

FIGURE 19: Erythemas 24 hours later the solutions are applied.
FIGURE 20: Erythemas 72 hours later the solutions are applied.

In figures 19 and 20 we can appreciate how the crythema caused by the SDS is not as intense in the case of the solution number 2 (SDS 2,5%+0,5% of LIPCUAT-4) in comparison to the one caused by the solution number 1 (SDS 2,5%).

The difference is more evident as we compare the erythema caused by the solution number 1 (SDS 2.5%) and the erythema caused by the solution number 3 (SDS 2.5%+2 % of LIPCUAT-4).

CONCLUSION: The nanovector (+) LIPCUAT-4 reduces the skin rash caused by the anionic surfactants.

<u>9) SKIN MOISTURE TEST</u>

Introduction: During the daily hair wash the lubricants are dragged during the rinsing after they are emulsified, producing hair and scalp dryness.

In the case of the hair, it looses elasticity as well as traction resistance, and it breaks more easily.

It deteriorates the skin barrier of the scalp and it produces dryness, exfoliation, and it irritates the skin producing a rash.

The lack of moisture weakens the hair.

Thus, it is important to keep the scalp hydrated and to provide it with the necessary lipids to restore its skin barrier.

Test's objective: to determine the LIPCUAT-4 ability to moisturize the scalp.

Materials and method applied:

Equipment used: CM 825 CORNEOMETER ®.

Origin: Germany

Procedure:

1)-Areas of 4 $cm^2$ are cleaned with a solution containing a 70% of ethanol on the scalp of 6 people, 3 of whom had normal hair, and 3 of whom had advanced hair loss.

2)-After 15 minutes, the relative moisturizing value is measured with the corneometer on each area.

3)-In 3 of each of these areas, 0.10 cc. of a solution containing a 5% of LIPCUAT-4 are applied.

4)-The other 3 areas are control areas in which distilled water is applied.

This is time 0.

5)-The values are measured every hour with the corneometer.

After 10 hours the results are averaged out, which will be found in the graph chart herein below.

[On the top of the graph it reads: SKIN MOISTURE TEST. On the left of the graph it reads: CORNEOMETER VALUES. On the bottom of the graph it reads: TIME IN HOURS]

FIGURE 21: from this chart we can infer that the solution containing a 5% of LIPCUAT-4 moisturizes the scalp up to a 100% above the control results.

10) TEST TO DETERMINE THE ABILITY OF THE STICKED LIPCUAT-6 TO ELIMINATE FREE RADICALES FROM THE HAIR AFTER RINSING

(Encapsulated nanovector with positive charge, Coenzyme Q10, sodium phosphate ascorbyl, Vitamin E)

**Introduction**: LIPCUAT-6 is an ANTIOXIDANT NANOVECTOR that has nano vesicles of a size that goes from 100 to 300 nm.

Each particle has a phospholipids membrane attached to a cationic polymer that provides density to the superficial positive charge as well as high substantivity (it sticks to the hair even after it is rinsed) to hair.

In its membrane it encapsulates and carries two hydrophobic actives that are anti-free radicals, antioxidants and stabilizers: Coenzyme Q10 and Vitamin E.

Inside of it, it has a liquid solution composed by sodium phosphate ascorbyl (hydrosoluble formula stabilized with vitamin C), which has a great antioxidant activity. It also has octyl methoxycinnamate, which is a sunscreen of a large spectrum that protects the capillary structure from the UV rays action.

Some of the structural amino acids that are part of the hair keratin, such as: cysteine, tyrosine, phenylalanine and tryptophan, have chromophores that are stimulated by UV rays. If the radiation is intense, there is molecular resonance, thus, double bonds are broken, producing free radicals and they are no longer neutral.

At the same time, the resultant free radicals attack and break the disulphure bonds, thus, the hair gets bleached, becomes more fragile and looses its elasticity and glow.

LIPCUAT-6 protects the hair from solar rays due to the fact that it contains a sunscreen and fights the free radicals due to the fact that the Coenzyme Q10, the sodium phosphate ascorbyl, and the Vitamin E are found within its components; therefore, it avoids hair deterioration.

**The objective of this study**: To appreciate the antioxidant activity of LIPCUAT-6. To value the ability of LIPCUAT-6 to eliminate free radicals as it sticks to the hair after applied and rinsed with plenty ofwater.

**Method:** the antioxidant activity is determined by the DDPH method, "DPPH Assay", modified. The DPPH method is simple and quick, and it allows to measure the antioxidant activity and how free radicals are intercepted. This peculiar characteristic is known as the free radical *scavenger* extintion phenomenon. The DPPH (2,2-diphenyl-1-picrylhydrazyl) has a peculiar characteristic that when in a solution containing alcohol it produces free radicals and, as a consequence, the molecule is electronically modified turning the solution into purple. The antioxidants react when in contact with the DPPH turning it into a stable colorless solution: diphenyl picryl hydrazine.

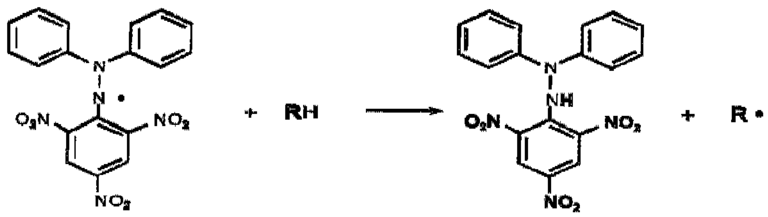

VIOLETA
DPPH 515 nm
RH: ANTIOXIDANTE
INCOLORO
DPPH 515 nm

FIGURE 22
Materials.
Shmiadzu spectrophotometer UV Model 1203
DPPH 2,2 diphenyl 1 pricrylhydrazyl (Fluka)
Absolut ethanol (Sintorgan)
Ascorbic acid (Prest)

LIPCUAT-6 ANTIOXIDANT NANOVECTOR
Composition:

| | |
|---|---|
| Purified phospholipds with soy lecithin | 10.00 grams % |
| Polyquaternium -16: | 10.00 grams % |
| Octyl methoxycinnamate. | 1.00 grams % |
| Sodium phosphate ascorbyl. | 0.50 grams % |
| Alpha tocopherol acetate: | 0.10 grams % |
| Coenzyme Q10: | 0.10 grams % |
| Deionized water | qs 100.00 grams % |

Procedure:

First step:

1) The hair locks are prepared: virgin hair without any chemical treatment of a 25 year old woman is used. Four locks of 10 cm long and of 2 g each are selected. The locks have a diameter that goes from 0.070 and 0.078 mm and are measured by means of a digital micrometer.

2) Preparation of the solution containing a 20% of DPPH: weight 10 mg (0.01 g) of DPPH 2,2 diphenyl 1 prierylhydrazyl (Fluka), put it into a flask of 100 ml and bring to volume with ethanol. Take 20 ml of this solution, put in a flask of 100 ml and bring to volume with distilled water (20% DPPH SOLUTION).

Second step:

a) The 4 locks are washed with ethanol during 10 minutes and afterwards are left to dry outdoors at room temperature.

b) They are immersed in a solution containing a 20% of DPPH during 24 hours

c) Rinse the 4 locks with plenty of water and dry them with hot air by means of a hair dryer.

d) Immerse 2 locks in a solution containing 0.5% of LIPCUAT-6 and immerse the other 2 locks in distilled water during 10 minutes.

e) Rinse the 2 locks immersed in the solution containing 0.5% of LIPCUAT'-6 separately with plenty of watery and dry with hot air by means of a hair dryer the other 2 locks immersed in distilled water.

f) Prepare 7 test tubes and tag them as follows:

2 TUBES: Hair treated with LIPCUAT-G 0.5%.

2 TUBES: Hair treated with distilled water.

1 TUBE: Control.

2 TUBES: Ascorbic acid 0.005 g.

g) Put each lock in its corresponding tube, in two of the tubes put 0.005 g of ascorbic acid.

h) Fill each tube with 10 ml of the solution containing 20% of DPPH and measure the absorbance with a spectrophotometer to 515 nm.
FIGURE 23: tube B is the tube control containing DPPH 20%.
Inside tube A we find the untreated lock.
Inside tube C we find the lock treated with the solution containing LIPCUAT-6.

i) Once the reaction takes place and the absorbance levels are measured, the following formula is applies in order to obtain the percentage levels of free radicals extinction (%ERL).

$$\%ERL = \frac{Abs_{control} - Abs_{Munstra}}{Abs_{control}} X\ 100$$

j) These values are put into a graph:

FIGURE 24: COMPARATIVE GRAPH OF THE ERL % OF THE LIPCUAT-6 STUCK TO THE HAIR VS. ASCORBIC ACID 0.005 g.

RESULTS: In this graph Figure 24, we can appreciate that the upper blue curve represents the percentage of the LIPCUAT-6's particles capacity to eliminate free radicals as they stick to the hair after being applied and rinsed with plenty of water.

The lower red curve represents the percentage of the free radical extintion on 0.005 g of ascorbic acid just diluted.

CONCLUSIONS:

a) LIPCUAT-6 has a great substantivity to the capillary libber due to the fact that it sticks to the ha ir, even after it is rinsed with plenty of water.

b) The LIPCUAT-6 particles stuck to 2 g of natural hair, after it is immersed in a 0.5% solution, and rinsed with plenty of water, they are able to extinct free radicals superior to 0.005 g of ascorbic acid..

c) LIPCUAT-6 has a great antioxidant and anti- free radical activity.

Interesting pharmacotechnic applications of the product of this invention:

In shampoos: in some cases it enhances the substantivity to the hair up to 100% of the incorporated actives compared to those shampoos in which the actives are incorporated in tube traditional way.

It reduces the irritability produced by the anionic tensoactives. It makes the hair more manageable and it protects the capillary fiber.

In gels and leave on conditioners and lotions: it allows fat soluble substances to be added without having the need to emulsify them with tensoactives that damage the hair and skin, for example: vitamin E, vitamin A, and coenzyme Q10.

Another interesting example are the sunscreens such as octocrylene, octyl methoxycinnamate, etc. Which are ideal to protect the hair from the UV rays.

In shower gels, it provides the skin with a velvety feeling once it is dry.

If antioxidant vitamins are added, they help to prevent the premature ageing of the skin.

## Claims

1. "NANOVECTOR WITH POSITIVE CHARGE SUBSTANTIVE FOR HAIR AND SKIN ", of the kind that can be applied directly on the skin or hair, or it may be added to the preparation of a cosmetic or *dermopharmaceutical* compositions in order to enhance the properties of said products; comprising empty particles dispersed in a water-based medium of the range of the nanometers; those particles have mainly the following components: a phospholipids membrane of any order and composition, and one or more quaternary polymers that provide it with a superficial positive charge, both components may be found in any concentration combination.

2. "NANOVECTOR WITH POSITIVE CHARGE SUBSTANTIVE FOR HAIR AND SKIN ", according to claim 1; wherein the phospholipids can either be from vegetable (mainly soy lethitin) or from animal (nervous tissue, eggs, etc.) sources or synthetic; those from vegetable source are generally composed by phosphatidylcholine, phosphatidylethanolamine, phosphatidylinositol, phosphatidylserine, fatty acids, etc; its concentration can go from 1 to 30%, more preferably from 5 to 15%.

3. "NANOVECTOR WITH POSITIVE CHARGE SUBSTANTIVE FOR HAIR AND SKIN ", according to claim 1; wherein the quaternary polymers may be either Polyquaternium 16, Polyquaternium 7 or the Polyquaternium 1, 2, 6, 10, 22, 28, 32, 39, 46 and 47; the concentration of the quaternary polymer(s) can go from 1 to 40%, more preferably from 5 to 15%.

4. "NANOVECTOR WITH POSITIVE CHARGE SUBSTANTIVE FOR HAIR AND SKIN ", according to claim 1; wherein the particles are able to carry lipophilic actives in its membrane and hydrophilic within its membrane.

5. "NANOVECTOR WITH POSITIVE CHARGE SUBSTANTIVE FOR HAIR AND SKIN ", according to claim 1; wherein the membrane may contain antioxidant substances, butyl hydroxy toluene (BHT), butyl hydroxy anisole (BHA), vitamin E, vitamin C, etc., in order to avoid the membrane and the carried actives to oxidize.

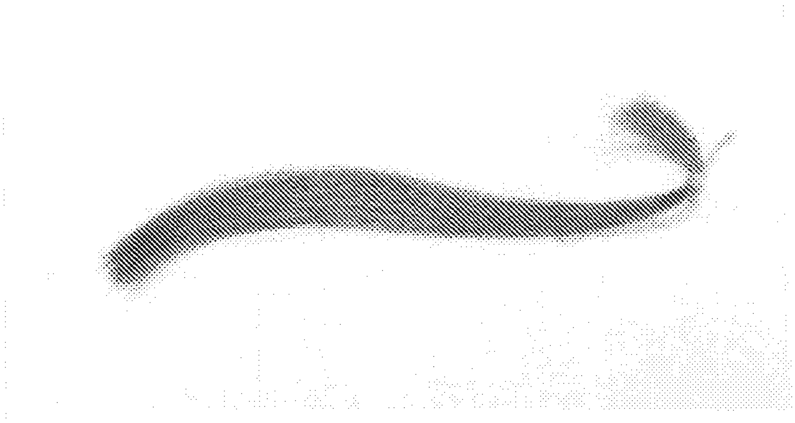

FIGURE 1: Hair treated with LIPCUAT. In this figure you can appreciate that the red coloring adheres to the hair, thus the presence of the cationic components on the capillary surface is confirmed.

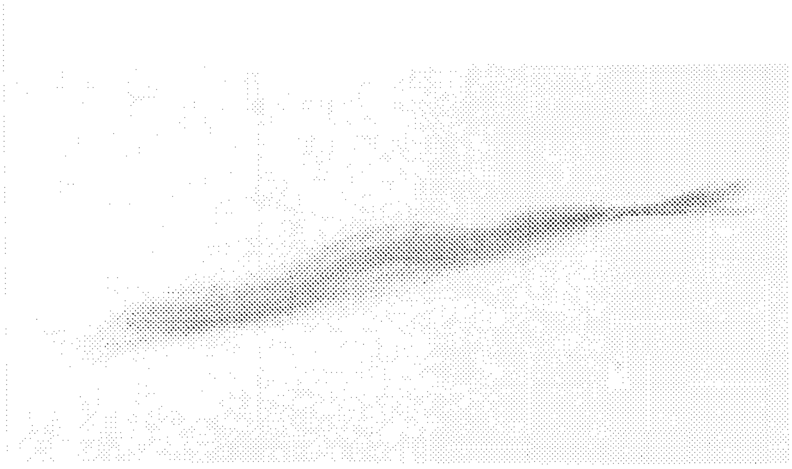

FIGURE 2: Control hair: not treated with LIPCUAT. The coloring does not adhere to the capillary fiber. The color of the hair is not altered due to the fact that there are no cationic components over the capillary fiber, thus the red coloring does not adhere.

CONCLUSION: THE LIPCUAT HAS A HIGH ABILITY TO STICK TO THE HAIR AND IS RESISTENT TO RINSING.

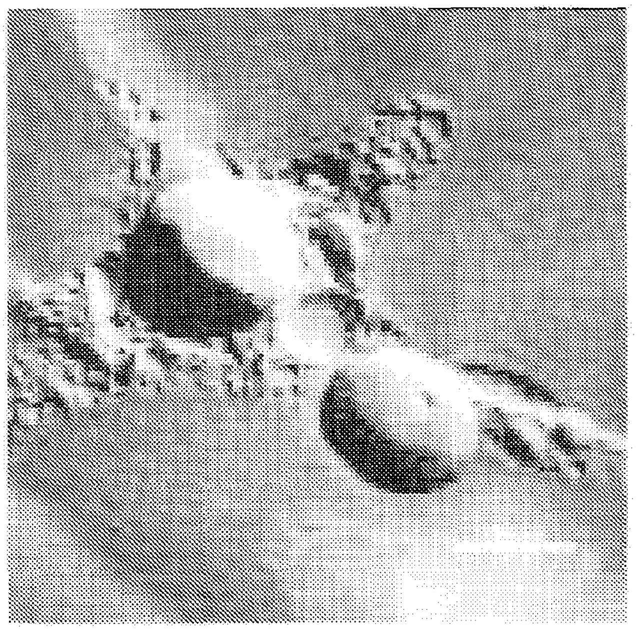

FIGURE 3. Microscopic view.

Technique: Transmission electron microscopy (TEM) (Zeiss EM902), afterwards a photograph of the cryofracture is taken with a digital Nikkon camera. This study is carried out in the *Universidad Nacional de Cuyo* [National University of the Cuyo], Faculty of Medical Science. *IHEM CONICET* [Histology and Embryology Institute of the city of Mendoza]. Av. Libertador 80. Centro Universitario. Parque Gral. San Martin (5500) Mendoza. Argentina.

Scanning electron microscope examination.

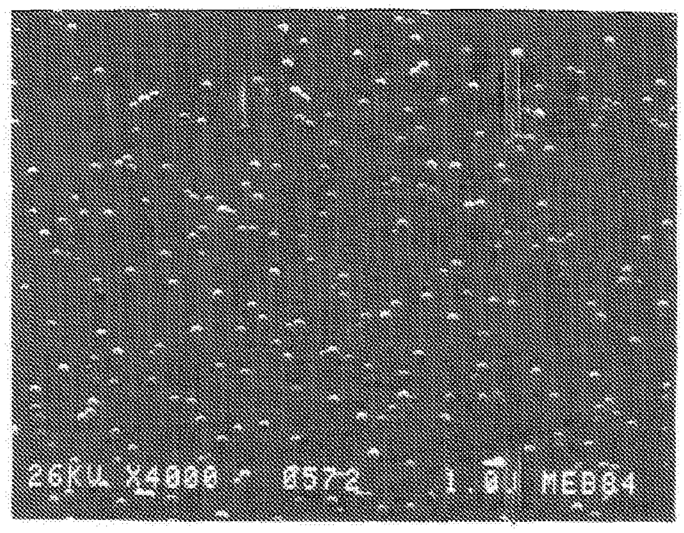

FIGURE 4: Scanning electron microscope examination. Carried out in the Faculty of Odontology.

The picture illustrates the Nanovectors (+) micro-fluidificated. As we compare the size of them with the reference bar (1 micron) we can appreciate that the average size is below the 300 nanometers.

4) STABLISHING THE LIPCUAT CHARGE BY MEANS OF ELECTROPHORESIS.

Procedure: a run with electrophoresis of agarose gel is carried out. Several LIPOSOMES with negative superficial charge (-) are scattered in the center and 3 lanes with LIPCUAT with positive superficial charge (+).

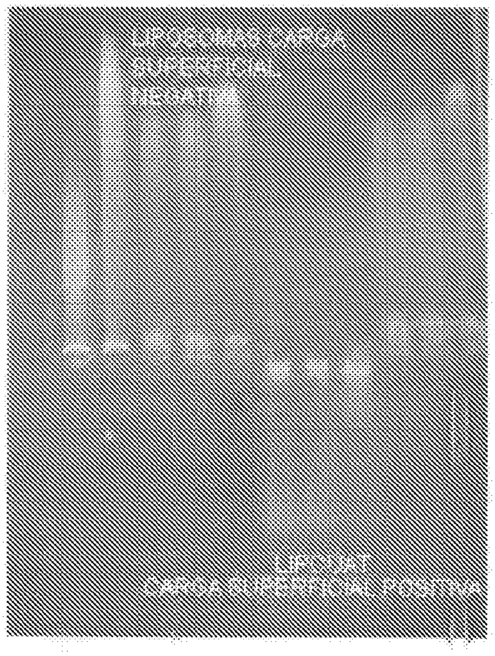


[On the top of the picture it reads: LIPOSOMES WITH NEGATIVE SUPERFICIAL CHARGE. On the bottom of the picture it reads: LIPCUAT WITH POSITIVE SUPERFICIAL CHARGE]

FIGURE 5. From this picture we can appreciate that the product object of this invention (LIPCUAT) migrates towards the anode charge (-). We also can appreciate that the migration

After 21 days, the last centimeters of the ends of each hair are observed with the digital microscope. Pictures of each one are taken, which are found herein below:

Lock – Exposed to the sun – with gel with a 5% of LIPCUAT.

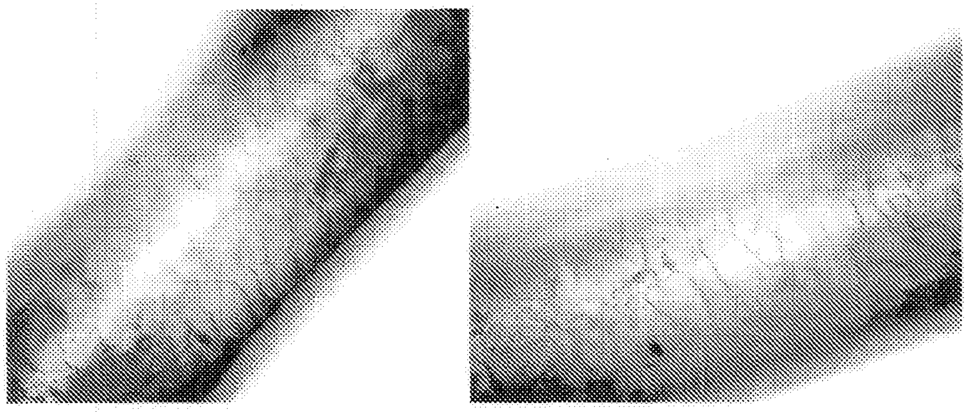

FIGURE 6: the hair protected with LIPCUAT does not show any modification in its cuticle, it is soft and has no imperfections. The hair maintains its elasticity. When touched, you have a soft feeling.

Lock - Exposed to the sun – with gel base.

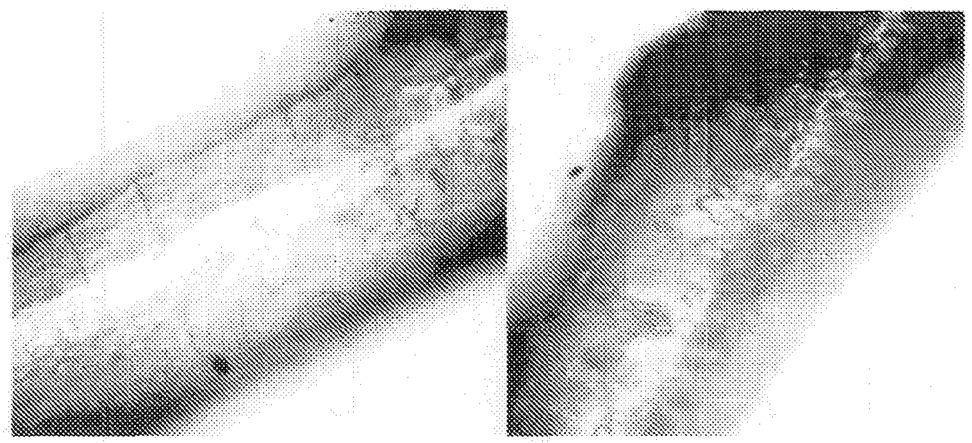

FIGURE 7: the hair with gel base, without any protection, is heavily affected by the solar rays, its cuticle is destroyed and its body is cracked as if it had been exposed to high temperatures or even burnt. The hair is fragile, rough and when touched, you have an unpleasant feeling.

Lock – without being exposed to the sun.

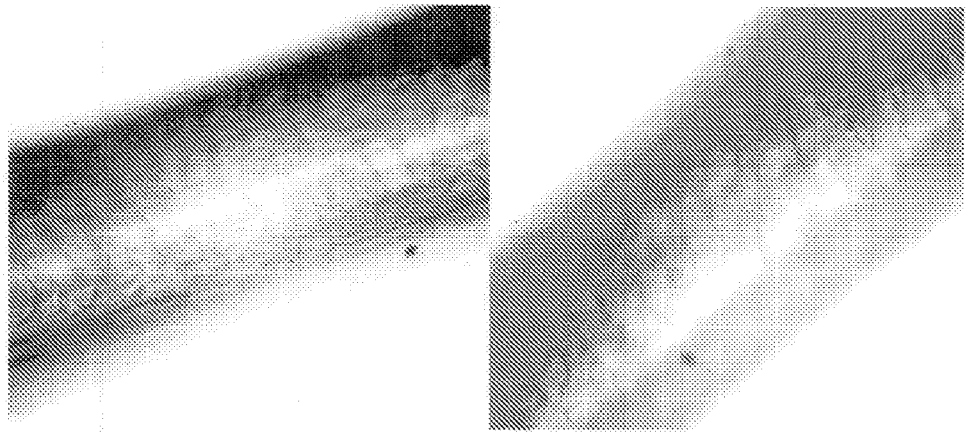

FIGURE 8: the hair has not been exposed to the sun and you can appreciate that its cuticle is intact, slightly lifted. When touched, it feels less rough that the hair without protection, but, it feels rougher than the lock protected by LIPCUAT.

CONCLUSION: the LIPCUAT is effective for hair protection, specially its ends, from solar radiations. It also conditions, maintains and improves the elasticity of the hair, it makes it softer and brighter at the same time.

6) TEST ON THE HAIR TRACTION RESISTENCE WHEN TREATED WITH LIPCUAT

Introduction: daily wash, chemical treatments, UV rays and time weaken the capillary fiber. This leads to the increase of hair loss, reduces its density and elasticity, etc.

The measurements carried out confirm that the more deteriorated and more mistreated the hair is the less resistance it has to traction.

Objective: to determine the ability of LIPCUAT to enhance the traction resistance of the hair once it is added to a shampoo.

Method: the hair is stretched until it is broken while its traction resistance is measured by means of a digital dynamometer. Locks rinsed with water are compared with those washed with shampoo and those washed with shampoo with LIPCUAT.

RESULTS

CURVE OF THE BREAK RESISTANCE:

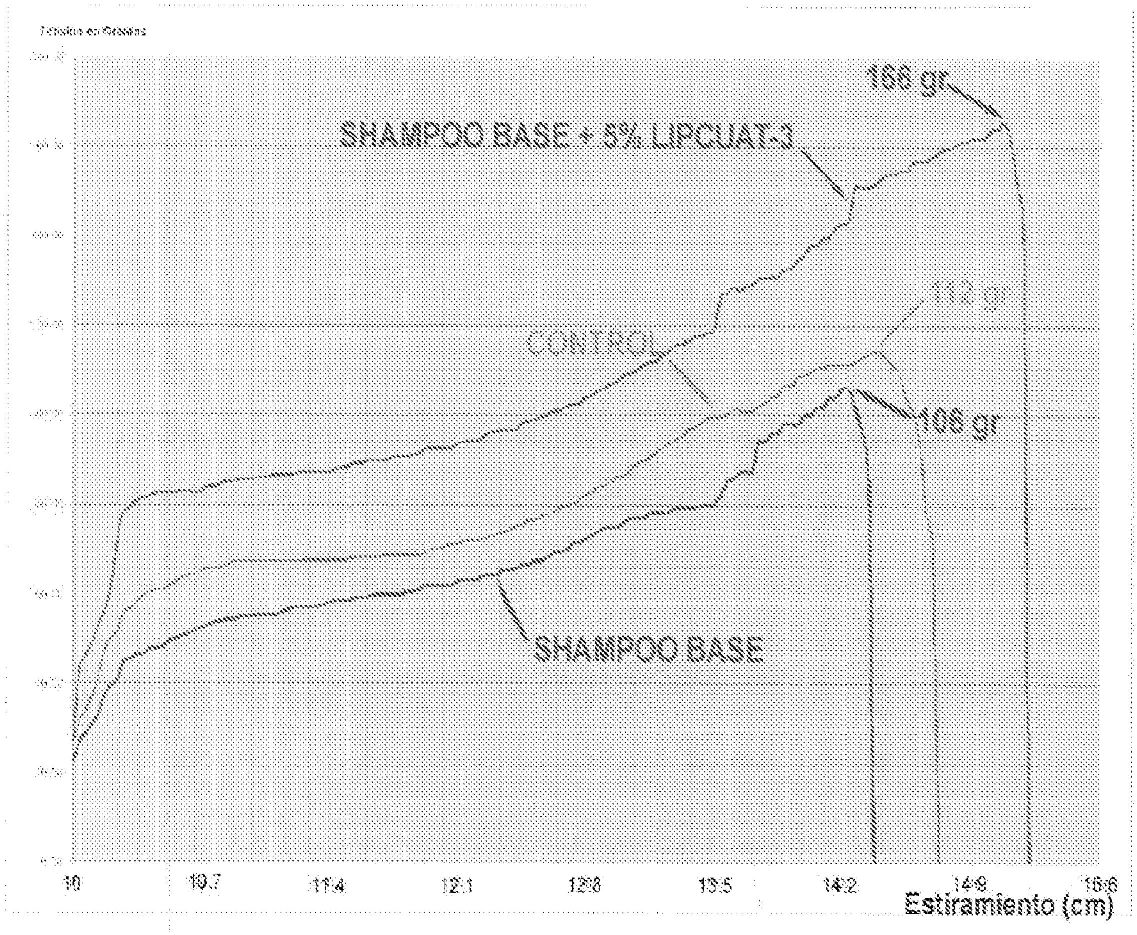


FIGURE 9: in the graphic we can appreciate that the hair treated with LIPCUAT is more resistant to tension (more charge) before it breaks, the product improves the resistance of the capillary fiber in comparison to the hair treated with the base shampoo.

BAR GRAPH:

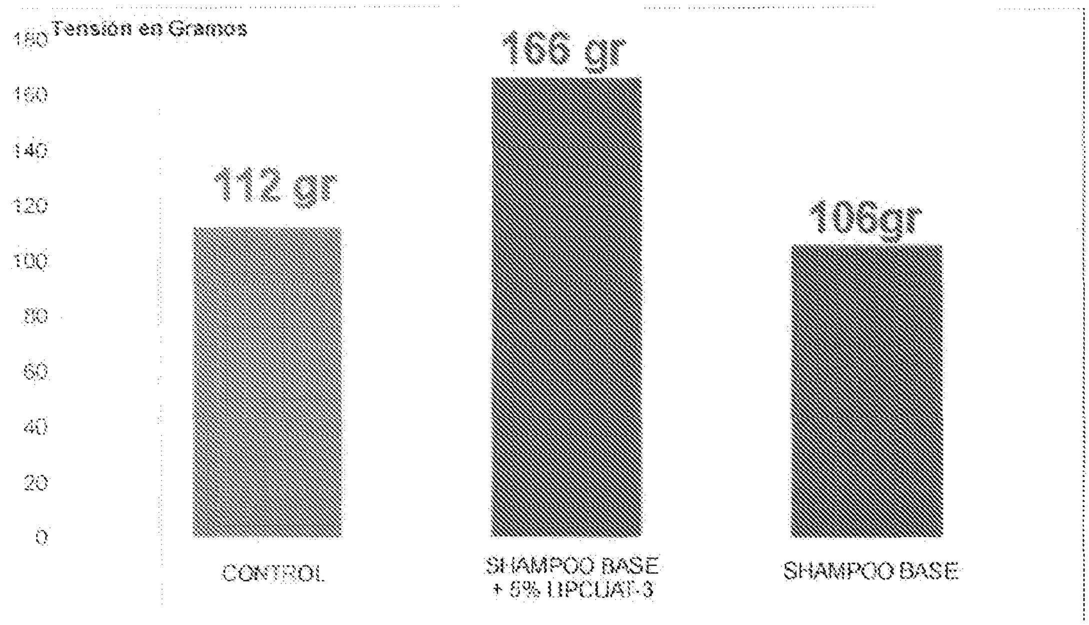


FIGURE 10: in the graph bar it is clearly shown that the resistance is improved in the hair treated with LIPCUAT compared to the control hair. We can also appreciate how the resistance is reduced with the base shampoo compared to the control.

CONCLUSION: The hair resistance to traction is enhanced by LIPCUAT. The capillary fiber becomes more resistant and it breaks less. From these results we can infer that LIPCUAT reduces hair loss during combing.

7) TEST ON THE SUBSTANTIVITY AND ON THE LIPCUAT LAYER FORMATION.

Objetive of the test:

To determine the LIPCUAT's substantivity (ability to adhere to the structure) with the hair, even after it has been rinsed.

To verify the ability to penetrate though the capillary structure pores.

To confirm the formation of a film over the surface as a consequence of the particles' fusion.

Method: the scanning electron microscope (SEM) is used in order to observe the capillary fiber and the LIPCUAT in different conditions.

d)- After 24 hours, the treated and untreated hairs are located on the glass dishes and when dry, a thin layer of gold is applied to them (metalize) in order to be observed through the electron microscope.

e)- The samples are observed through the scanning electron microscope and digital photographs are taken.

Test 4: A drop of LIPCUAT is put on the glass dish and it is let to dry at room temperature outdoors

After 24 hours, the treated and untreated hairs are located on the glass dishes and when dry, a thin layer of gold is applied to them (metalize) in order to be observed through the electron microscope.

Digital photographs are taken.

Test 1:

Hair A: without treatment.

Hair B: The hair is immersed in LIPCUAT for 5 minutes; it is let to drain and dry outdoors.

The hairs are observed 24 hours later with the scanning electron microscope SEM.

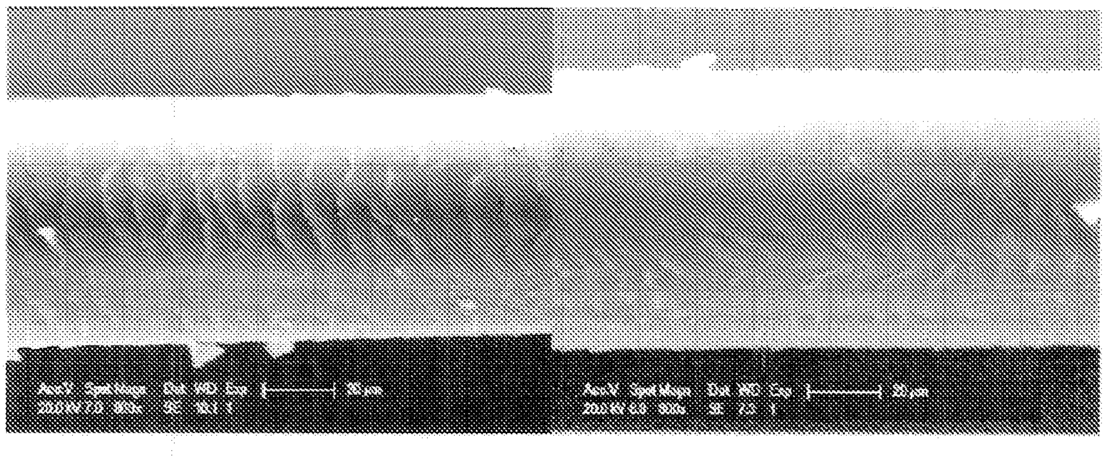

FIGURE 11: Hair A (control) Hair B (treated with LIPCUAT)

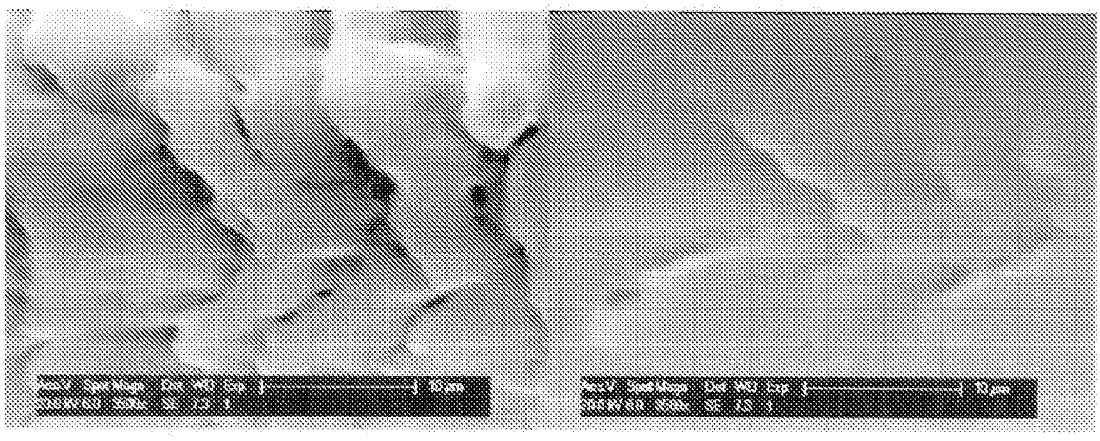

FIGURE 12: Hair A (cuticle detail) Hair B (cuticle detail)

Test 2

Hair A: Without treatment.

Hair C: The hair is immersed in a solution with 5% of LIPCUAT for 5 minutes, afterwards it is left to drain and dry outdoors.

The hairs are observed 24 hours later with the scanning electron microscope SEM.

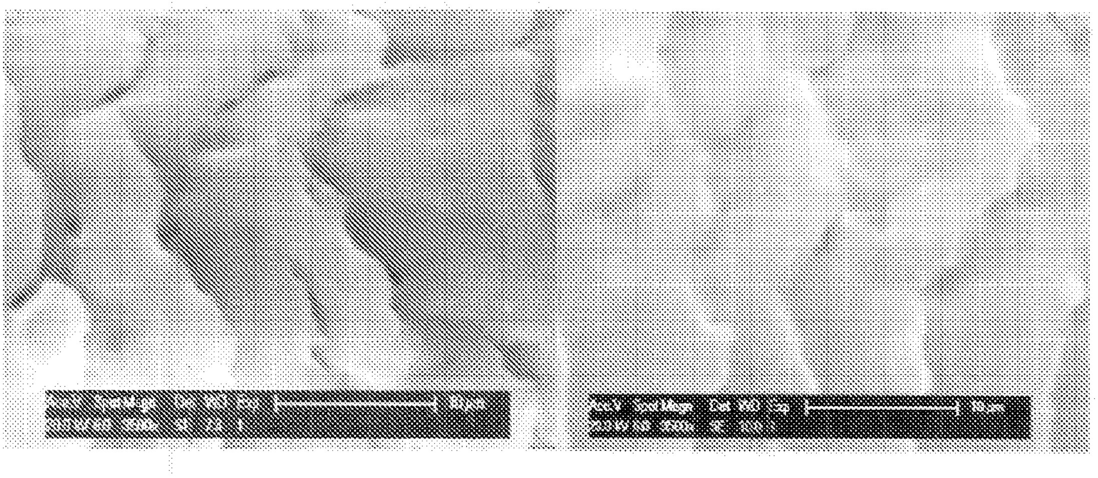

FIGURE 13: Hair A (cuticle detail) Hair C (cuticle detail)

Test 3:

Hair A: without treatment.

Hair D: The hair is immersed in a solution with 1% of LIPCUAT for 5 minutes; afterwards it is rinsed with plenty of water and let to drain and dry outdoors.

The hairs are observed 24 hours later with the scanning electron microscope SEM.

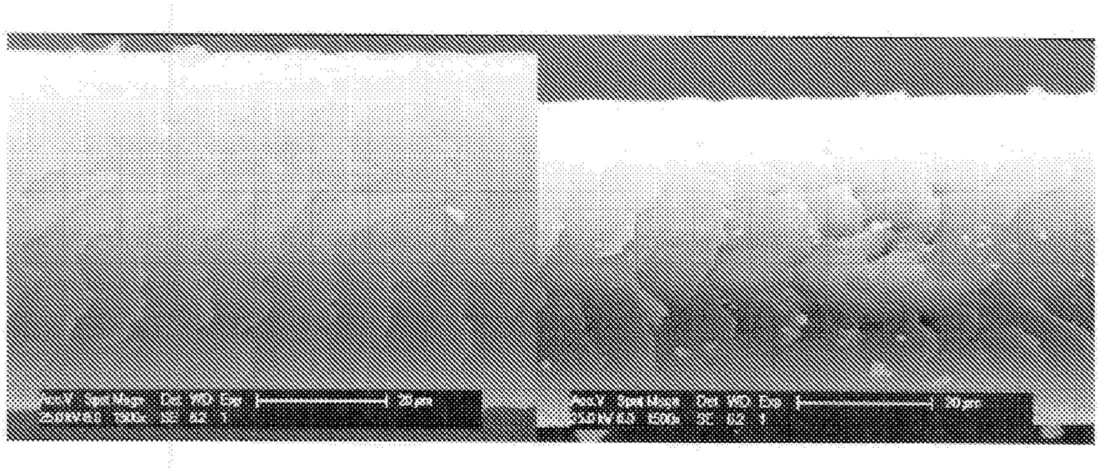

FIGURE 14: Hair A (control) Hair D (treated with LIPCUAT)

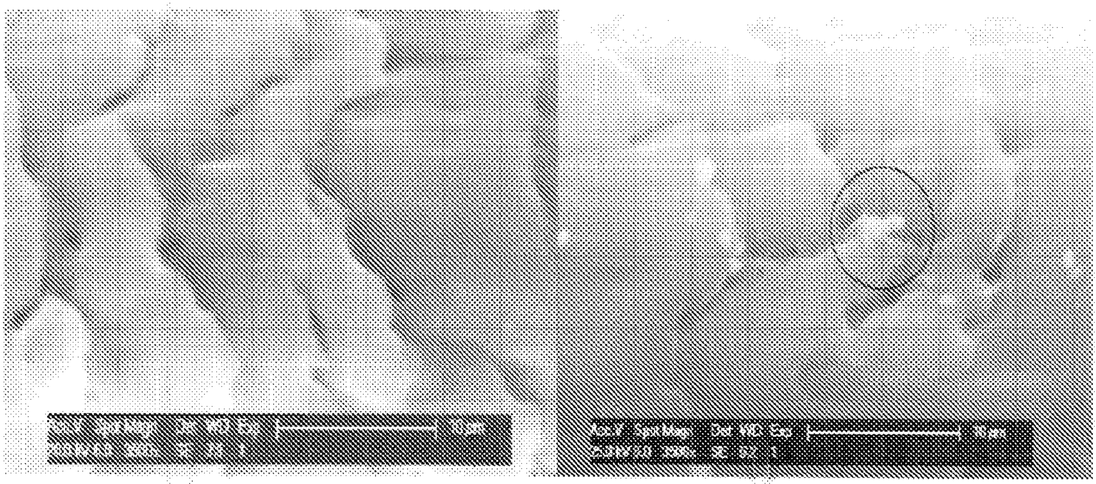

FIGURE 15: Hair A (cuticle detail) Hair D (cuticle detail)

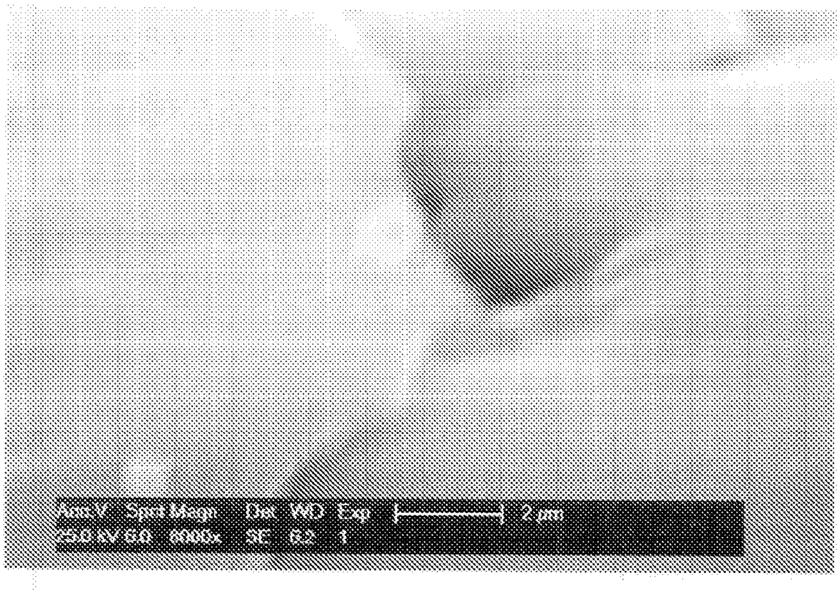


FIGURE 16: Hair D (cuticle detail)

Test 4:

A drop of LIPCUAT is put on the glass dish and is left to dry at room temperature.

The drop is observed 24 hours later with the scanning electron microscope SEM.

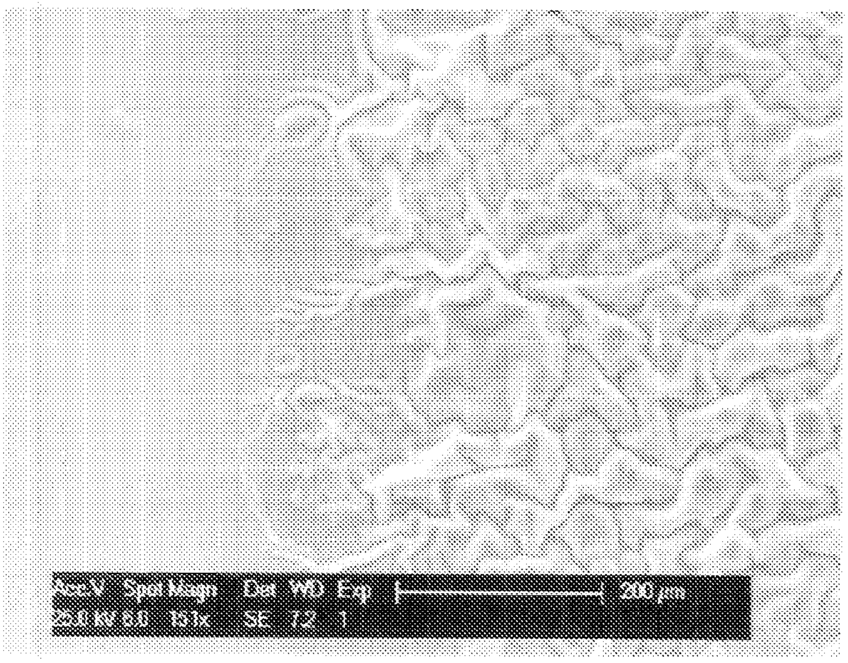


FIGURE 17: Fusioned LIPCUAT particles creating a film on the glass dish.

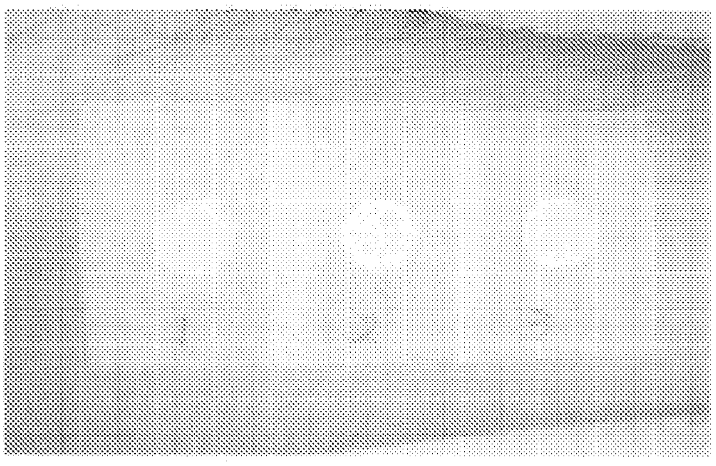


FIGURE 18: FINN chambers on the forearm.

e) These are left for 24 hours and then removed.

f) The area is cleaned with a solution containing a 70% of ethanol.

g) After 30 minutes, the tested areas are evaluated.

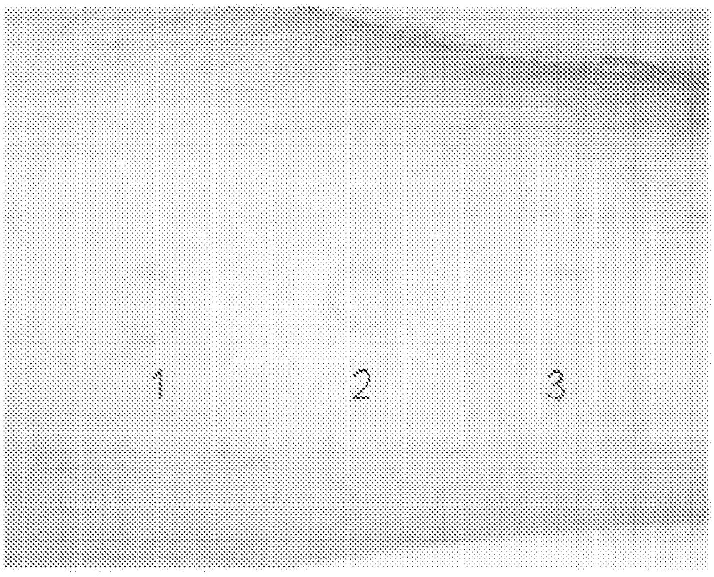


FIGURE 19: Erythemas 24 hours later the solutions are applied.

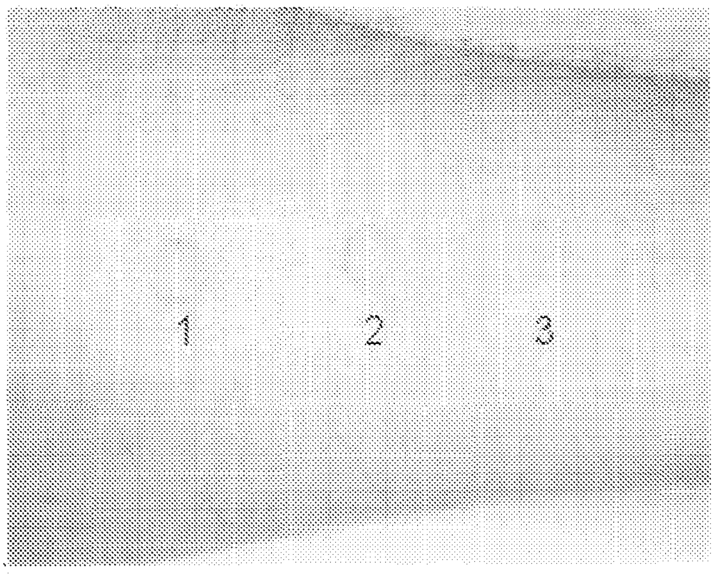


FIGURE 20: Erythemas 72 hours later the solutions are applied.

In figures 19 and 20 we can appreciate how the erythema caused by the SDS is not as intense in the case of the solution number 2 (SDS 2,5%+0,5% of LIPCUAT-4) in comparison to the one caused by the solution number 1 (SDS 2,5%).

The difference is more evident as we compare the erythema caused by the solution number 1 (SDS 2.5%) and the erythema caused by the solution number 3 (SDS 2.5%+2 % of LIPCUAT-4).

CONCLUSION: The nanovector (+) LIPCUAT-4 reduces the skin rash caused by the anionic surfactants.

9) SKIN MOISTURE TEST

Introduction: During the daily hair wash the lubricants are dragged during the rinsing after they are emulsified, producing hair and scalp dryness.

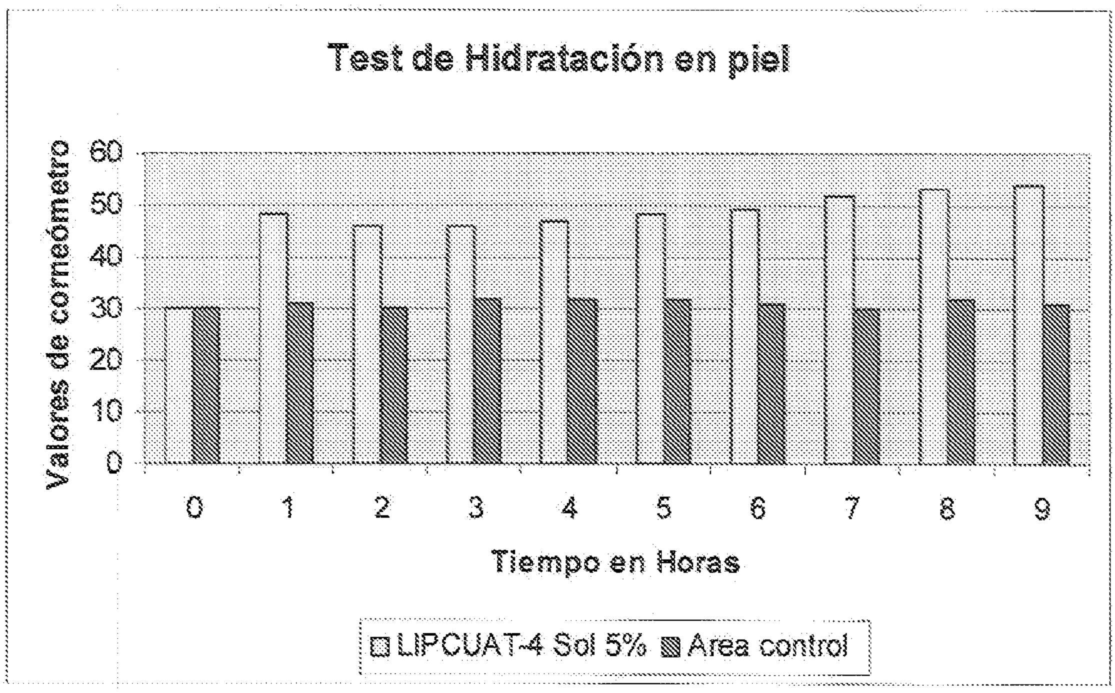


[On the top of the graph it reads: SKIN MOISTURE TEST. On the left of the graph it reads: CORNEOMETER VALUES. On the bottom of the graph it reads: TIME IN HOURS]

FIGURE 21: from this chart we can infer that the solution containing a 5% of LIPCUAT-4 moisturizes the scalp up to a 100% above the control results.

10) TEST TO DETERMINE THE ABILITY OF THE STICKED LIPCUAT-6 TO ELIMINATE FREE RADICALES FROM THE HAIR AFTER RINSING

(Encapsulated nanovector with positive charge, Coenzyme Q10, sodium phosphate ascorbyl, Vitamin E)

**Introduction:** LIPCUAT-6 is an ANTIOXIDANT NANOVECTOR that has nano vesicles of a size that goes from 100 to 300 nm.

2 TUBES: Hair treated with distilled water.

1 TUBE: Control.

2 TUBES: Ascorbic acid 0.005 g.

g) Put each lock in its corresponding tube, in two of the tubes put 0.005 g of ascorbic acid.

h) Fill each tube with 10 ml of the solution containing 20% of DPPH and measure the absorbance with a spectrophotometer to 515 nm.

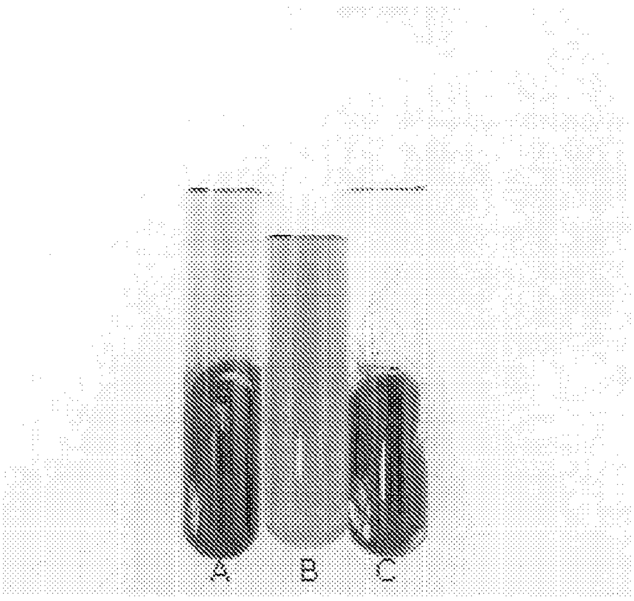


FIGURE 23: tube B is the tube control containing DPPH 20%.

Inside tube A we find the untreated lock.

Inside tube C we find the lock treated with the solution containing LIPCUAT-6.

i) Once the reaction takes place and the absorbance levels are measured, the following formula is applies in order to obtain the percentage levels of free radicals extinction (%ERL).

$$\%ERL = \frac{Abs_{control} - Abs_{Muestra}}{Abs_{control}} X\ 100$$

j) These values are put into a graph:

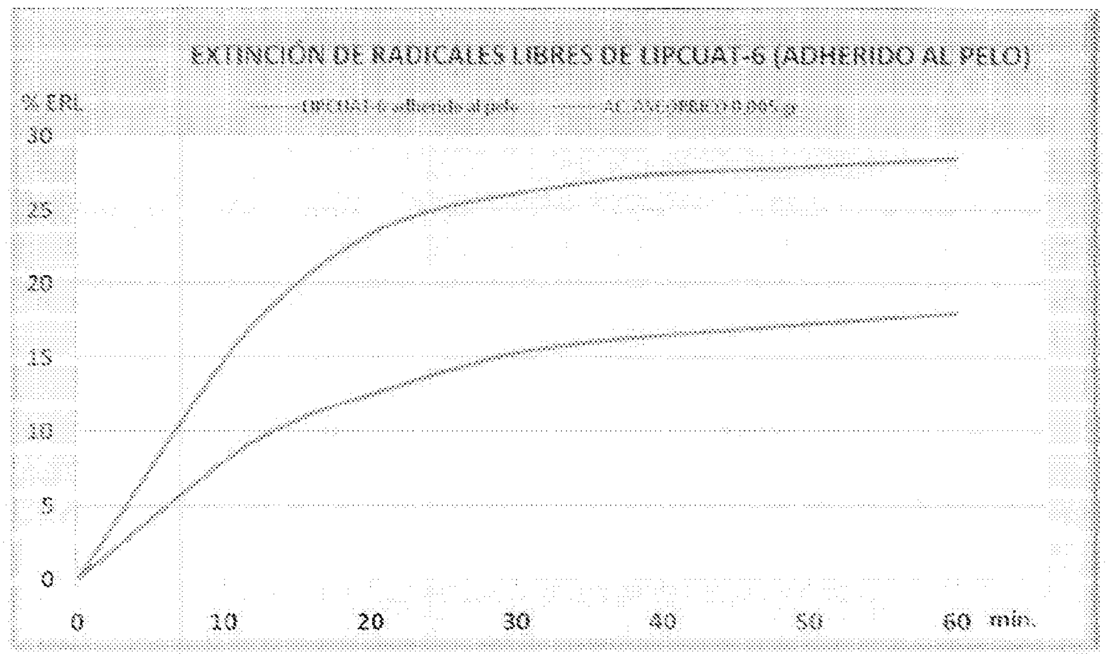


FIIGURE 24: COMPARATIVE GRAPH OF THE ERL % OF THE LIPCUAT-6 STUCK TO THE HAIR VS. ASCORBIC ACID 0.005 g.

RESULTS: In this graph Figure 24, we can appreciate that the upper blue curve represents the percentage of the LIPCUAT-6's particles capacity to eliminate free radicals as they stick to the hair after being applied and rinsed with plenty of water.

The lower red curve represents the percentage of the free radical extintion on 0.005 g of ascorbic acid just diluted.

CONCLUSIONS:

a) LIPCUAT-6 has a great substantivity to the capillary fiber due to the fact that it sticks to the ha ir, even after it is rinsed with plenty of water.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5328628 A

### Non-patent literature cited in the description


- *Revista Dermatologia Venzolana,* 1995, vol. 33 (1
- *Dra. Beatriz Clares Naveros' Doctoral Thesis,* 2003
- Journal of Oleo Science. Japan Oli Chemists' Society, 2007, 35-42